# EUROPEAN PATENT SPECIFICATION

(11) **EP 1 313 511 B1**
(45) Date of publication and mention of the grant of the patent: **30.11.2005**
(21) Application number: 01971928.5
(22) Date of filing: 17.08.2001
(51) Int. Cl.: A61K 47/44, A61K 31/565, A61P 5/26

(54) **TESTOSTERONE ESTER FORMULATION FOR HUMAN USE**
TESTOSTERON-ESTER FORMULIERUNG ZUR ANWENDUNG AM MENSCHEN
FORMULATION D'UN ESTER DE TESTOSTERONE POUR LE TRAITEMENT DE L'HOMME

(30) Priority: 23.08.2000 EP 00202939
(43) Date of publication of application: 28.05.2003
(73) Proprietor: Akzo Nobel N.V., 6824 BM Arnhem (NL)
(72) Inventor: DE NIJS, Henrik, NL-5343 XC Oss (NL); KERSEMAEKERS, Wendy, Margaretha, NL-6531 RB Nijmegen (NL); SCHUTTE, Sephanus, Cornelis, NL-6861 EJ Oosterbeek (NL); VAN BRUGGEN, Johann, Gerhard, Cornelis, NL-5141 EN Waalwijk (NL)
(74) Representative: van Wezenbeek, Petrus M.G.F.
(86) International application number: PCT/EP2001/009569
(87) International publication number: WO 2002/015938

(56) References cited:
- EP-A- 0 303 306
- GB-A- 465 331
- GB-A- 1 567 515
- US-A- 4 071 623
- US-A- 4 220 599
- AGMO ANDERS ET AL: "Dopamine and sexual behavior in the male rabbit." PHARMACOLOGY BIOCHEMISTRY AND BEHAVIOR, vol. 55, no. 2, 1996, pages 289-295, XP000979029 ISSN: 0091-3057 cited in the application
- BEHRE H M ET AL: "Intramuscular injection of testosterone undecanoate for the treatment of male hypogonadism: Phase I studies." EUROPEAN JOURNAL OF ENDOCRINOLOGY, vol. 140, no. 5, May 1999 (1999-05), pages 414-419, XP002195484 ISSN: 0804-4643 cited in the application
- WU FREDERICK C W ET AL: "Oral progestogen combined with testosterone as a potential male contraceptive: Additive effects between desogestrel and testosterone enanthate in suppression of spermatogenesis, pituitary-testicular axis, and lipid metabolism." JOURNAL OF CLINICAL ENDOCRINOLOGY & METABOLISM, vol. 84, no. 1, January 1999 (1999-01), pages 112-122, XP002195442 ISSN: 0021-972X cited in the application
- KAMISCHKE AXEL ET AL: "Intramuscular testosterone undecanoate with or without oral levonorgestrel: A randomized placebo-controlled feasability study for male contraception." CLINICAL ENDOCRINOLOGY, vol. 53, no. 1, July 2000 (2000-07), pages 43-52, XP002195485 ISSN: 0300-0664 cited in the application
- VERHEUL H A M ET AL: "EFFECTS OF SEX GONADECTOMY AND SEVERAL STEROIDS ON THE DEVELOPMENT OF INSULIN-DEPENDENT DIABETES MELLITUS IN THE BB RAT" CLINICAL AND EXPERIMENTAL IMMUNOLOGY, vol. 63, no. 3, 1986, pages 656-662, XP000979005 ISSN: 0009-9104 cited in the application
- ANDERSON R.A.: "Prospects for hormonal contraception in the male." REPRODUCTIVE MEDICINE REVIEW, (2000) 8/2 (127-142). , 18 October 2000 (2000-10-18), XP008002304

## Description

### Field of the invention

The invention is in the field of the treatment of androgen-insufficiency related disorders, by the inducement into a human being of an adequate level of testosterone. The invention particularly pertains to a method of treatment in which a desired level of testosterone is induced by the administration of a medicament or, otherwise, to the manufacture of a medicament in such a treatment, wherein the medicament is a pharmaceutical formulation in the form of an oily solution for injection comprising testosterone decanoate dissolved in a pharmaceutically acceptable oily medium.

### Background art

Testosterone decanoate has been known as a potential therapeutic compound administered in animal trials since the thirties of the 20^{th} century. Thus, in GB 465,331 (published 1937), testosterone capric acid (decanoic acid) ester is disclosed among several other testosterone esters. Other publications related to animal testing involving testosterone decanoate are Verheul et al. (1986) and Agmo et al. (1996).

Verheul, H.A.M. et al., *Effects of sex, gonadectomy and several steroids on the development of insulin-dependent diabetes mellitus in the BB rat,* Clin. Exp. Immunol (1986) **63**, 656-662 describes an investigation into the question of whether several sex and steroid related factors, such as treatment with several steroids, including testosterone decanoate, affect the development of diabetes mellitus in BB rats, *quod non.* Ågmo, A. et al., *Dopamine and sexual behavior in the male rabbit* in Pharmacology Biochemistry & Behavior, Vol. 55, No.2, pp. 289-295 (1996) describes the administration of testosterone decanoate as one of several different methods of preparing certain rabbits so as to have suitable test animals for investigating the influence of dopamine on sexual behavior. Particularly, the testosterone decanoate treatment serves to provide test animals maintaining a low but stable level of sexual behaviour.

Despite this long-standing use in science, the compound per se has never reached a stage of use in human therapy in practice. Also a recent paper by Kamischke et al., in summarizing the state of the art with regard to esters of testosterone, mentions testosterone undecanoate and testosterone buciclate as being suitable in practice for use in male contraception, and is silent on testosterone decanoate (Axel Kamischke et al., Clinical Endocrinology (2000), 53, 43-52, see page 43). Other background publications on male contraception include papers by M. Cristina Meriggiola et al. (Fertility and Sterility Vol. 68, No. 5 (1997), pp 844-850 and Human Reproduction vol. 13 no. 5, pp. 1225-1229, 1998) which relate to male contraception by treatment with the anti-androgenic progestagen cyproterone acetate and either testosterone undecanoate or testosterone enanthate. Testosterone enanthate is also known as the androgen component in studies involving the progestagen desogestrel (*inter alia* in Frederick C. W. Wu et al., Journal of Clinical Endocrinology and Metabolism 84 (1), pp 112-122). None of these papers relate to using testosterone decanoate. One recent reference to testosterone decanoate is WO 97/41865, in which the compound is generally included in a broad range of summed-up esters of testosterone. In some patent documents originating from the seventies (BE 855163, US 4,220,599 (DE 2508615), US 4,071,623) testosterone esters of a chain length typically including decanoate are disclosed for oral administration. No teaching is provided on parenteral administration. From these disclosures at any rate the undecanoate would be considered as better suitable for oral administration. Indeed, testosterone undecanoate rather than the decanoate has become available as a preparation for oral use (marketed *inter alia* under the tradename Andriol® in several countries).

Still, the compound testosterone decanoate is not entirely devoid of practical utility, as a formulation of the above-identified type, i.e, one in the form of an oily solution for injection comprising testosterone decanoate dissolved in a pharmaceutically acceptable oily medium, is known, and is available on the market in a great number of countries worldwide, in many cases under the name of Sustanon®250. Sustanon®250 is an oily solution comprising four esters of testosterone, viz. testosterone propionate, testosterone phenylpropionate, testosterone isocaproate and testosterone decanoate. Each of the esters having a different pharmacokinetic profile, all of them have been considered essential for obtaining both a rapid onset (the smaller esters) and a long duration of action (the decanoate).

As background art, besides Sustanon®250, it can be mentioned that H.M.M. Behre and E. Nieschlag have published a chapter on comparative pharmacokinetics of testosterone esters in *Testosferone*, Chapter 11, pages 329-348 (1998). Even in this standard text on testosterone esters no information on the independent use of testosterone decanoate is given

### Summary of the Invention

It has now been found, and surprisingly so, that - contrary to the practice of including testosterone decanoate only in a judicious mixture of esters - testosterone decanoate alone provides an onset of action and a duration of action at least comparable to that of the mixture. This has led to the present invention, which resides in omitting three of the four esters as compared to the known mixture and thus provides a formulation of the above-indicated type, characterized in that testosterone decanoate is the sole ester of testosterone present in the oily medium.

### Detailed Description of the Invention

Testosterone decanoate being a compound which in effect serves to administer the male hormone testosterone, the formulation of the invention can be used in the treatment of human androgen-insufficiency related disorders. In the context of the invention, the term "androgen insufficiency" is to be understood to pertain to all kinds of diseases, disorders, and symptoms in which a male or a female suffers from too low a testosterone level, such as in hypogonadal men. In particular, the androgen insufficiency to be treated by the formulation of the invention is the reduction of the testosterone level which a human male incurs as a result of age (the formulation of the invention is then used for male hormone replacement therapy), or when he is subject to male contraception. In the context of male contraception, the formulation of the invention especially serves to neutralize the effect of regimens of male hormone contraception in which a sterilitant such as a progestagen or LHRH (luteinizing hormone releasing hormone) is administered regularly, *e.g*. daily, or it is used as the sole male contraceptive substance.

The formulation of the invention can be prepared by dissolving testosterone in a suitable amount of an oily medium, such as arachis oil, oleic acid, castor oil, and the like, including mixtures of oils. The amount of testosterone that can be dissolved differs per chosen medium, but will generally be within the range of from 100-400 mg/ml. The preferred oil is castor oil. Not only does this lead to a favorable overall stability (no or little crystallization and no re-esterification of testosterone), it particularly has an unexpectedly good release profile, with burst staying within the therapeutic window. Moreover it displays a good solubility of testosterone decanoate, the concentration thereof preferably being up to 200 mg/ml.

Additives common to injection fluids can be added to the solution if desired. Suitable additives are known to the person skilled in the art. Possible additives include liquids that serve to lower the viscosity of the fomulation, e.g. benzyl alcohol, benzyl benzoate, or benzyl propionate. Because of the way of administration, through injection, it is preferred to have as few additives in the solution as possible. This is customary in the art of making injection fluids, and does not require elucidation here.

The invention also pertains to the use of testosterone decanoate for the manufacture of a medicine in the treatment of human androgen-insufficiency disorders wherein testosterone decanoate is the sole androgen. Particularly, it has been found that testosterone decanoate, as the sole androgen, can be used advantageously in male contraception. This is notably so in a contraceptive regimen wherein the progestagen etonogestrel is employed to provide azoospermia, and testosterone decanoate is used to provide the required androgen supplementation. This combination of progestagen and androgen provides a relatively rapid onset of azoospermic effect, and provides a steady level of testosterone. In the context of the invention, azoospermia is considered to be found if 90% or more of the men receiving treatment exhibit azoospermia.

After intramuscular administration, testosterone levels will increase rapidly to high normal levels, with a peak (ca. 20-30 nmol/L) during the first few days. This comes as a surprise in view of what is known from Sustanon®250, namely that testosterone decanoate serves to attain prolonger duration of action, while the three shorter-chained esters serve to shift the onset of action to an earlier point in time. In the product of the invention, after the first few days, the testosterone levels will gradually decline to low physiological levels (ca. 12 nmol/L) after four or eight weeks, depending on the dose.

The invention is particularly well-suited to treat androgen-insufficiency which occurs as a result of deliberate progestagen-induced azoospermia (male contraception), notably when the progestagen is etonogestrel.

A preferred male contraceptive regimen is provided for by a kit (a drug delivery system) comprising a formulation of testosterone decanoate as described above, and etonogestrel in the form of oral daily tablets having a strength of 150 - 600 µg etonogestrel, preferably 150-300 µg. Alternatively, the etonogestrel is administered by means of one or more subcutaneous implants to release 40 to 200 µg etonogestrel per day, preferably 120 to 180 µg. Comparable implants are known from female contraception, e.g., under the name of Implanon®. For the manufacture of such implants, reference is made to EP 303 306. For a preferred contraceptive regimen, in the case of oral administration of etonogestrel, the dose in men will be approximately four and two times as high as that for desogestrel in the female OCs Cerazette® (75 µg desogestrel) and Marvelon® (150 µg desogestrel) respectively. When it comes to etonogestrel implants, preferably three times the release of progestagen is used as compared to the amounts administered to women, viz, three Implanon® 4 cm implants. More preferably two implants are used, each having 1,5 times the length of Implanon, or one single implant of more restricted length (e.g. 4-5 cm) providing a higher release. The implants are intended to be applied and removed yearly, although a longer action is not excluded. The androgenic component of the regimen according to the invention is preferably an injection of 300 mg to 700 mg, and preferably 400 to 600 mg testosterone decanoate given once per four weeks to eight weeks, and preferably once per six weeks. It is most preferred to give 600 mg once per six weeks, or 400 mg per four weeks. If desired, formulations can be provided which contain additives so as to provide a reduced and more prolonged level of testosterone (e.g. 800 to 1000 mg per 12 weeks using, e.g. a substantial amount of additive, such as 50% by weight of benzyl benzoate). This is formulation practice well-known to the skilled person. The drug delivery system according to the invention will preferably contain appropriate instructions as to the regimen of administration to be used in the provided method of treatment.

An unexpected advantage of this testosterone treatment is the relatively rapid onset of action, as displayed by the short time to reach a therapeutic level, much faster than could be expected on the basis of the common general knowledge of Sustanon®250.

In the contraceptive regimens based on etonogestrel combined with TD, azoospermia can be achieved well within 24 weeks, or even in a duration comparable to that in the case of vasectomy, which generally is 12-16 weeks.

The invention will be further illustrated with reference to the Examples which follow.

### Example 1 (preparation of solution)

Solutions of 200 mg/ml testosterone decanoate in castor oil are prepared by dissolving 1000 g of testosterone decanoate in about 3,5 L of the solvent at 50°C. Thereafter, solvent is added up to 5L. A clear solution is obtained, without visible particles of undissolved matter. After filtration the solution is ready for use.

### Example 2 (test in hypogonadal men)

To eight hypogonadal men an intramuscular injection of 500 mg testosterone decanoate in 2 ml arachis oil is given. Testosterone levels are measured at regular intervals for a period of 50 days. The results show a rapid onset of action in that an average therapeutic level of testosterone of 25 nmol/l is reached within 1.5 day (mean value), peak level is reached within 5 days, and a therapeutic level of more than 10 nmol/l is seen up to 30 days.

### Example 3 (test in hypogonadal men)

To eight hypogonadal men an intramuscular injection of 400 mg testosterone decanoate in 2 ml castor oil is given. Testosterone levels are measured at regular intervals for a period of 50 days. The results show a rapid onset of action in that an average therapeutic level of testosterone of 25 nmol/l is reached within 2 days peak level is reached within 8 days, and a therapeutic level of more than 10 nmol/l is seen up to 42 days (all mean values).

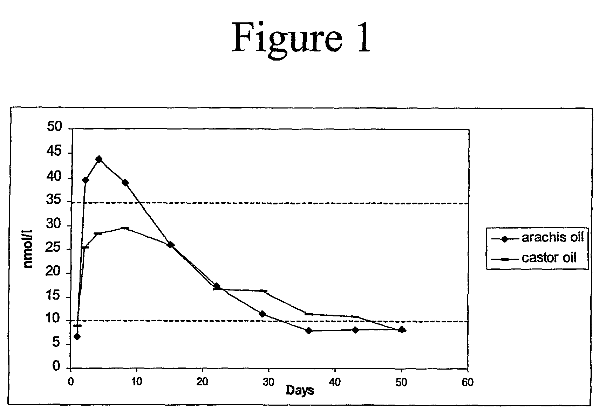

In Figure 1, the results of Examples 2 and 3 are presented in a graph. On the x-axis the number of days after administration is indicated, on the y-axis the determined blood levels of testosterone.

### Comparison

The results attained in the foregoing Examples are compared with the results of (A) Sustanon®250, 250 mg in 1 ml of arachis oil (J.A. Cantrill et al., Clin. Endocrinol. 1984;21;97-107, p102), and with testosterone undecanoate, 1000 mg in (B) 4 ml of castor oil and (C) two times 4 ml of tea seed oil (H.M.M. Behre et al., European Journal of Endocrinology (1999) **140** 414-419. The results are depicted in the table below. The formulations of the invention unexpectedly provide an onset of action comparable to that of Sustanon®250 and more rapid than testosterone undecanoate.

**Table**

| Preparation | Onset of action (25 nmol/l level) | Duration of action (> 10 nmol/l) |
|---|---|---|
| Example 2 | 1.5 days | 30 days |
| Example 3 | 2 days | 42 days |
| (A) | 1.5 days | 21 days |
| (B) | 7 days | 42 days |
| (C) | 6 days | 50 days |

## Claims

1. The use of a testosterone ester for the manufacture of a medicament to treat human androgen-insufficiency disorders, wherein the medicament is a pharmaceutical formulation in the form of an oily solution for injection comprising testosterone decanoate dissolved in a pharmaceutically acceptable oily medium, **characterized in that** testosterone decanoate is the sole ester of testosterone present in the oily medium.

2. A pharmaceutical formulation as referred to in claim 1, **characterized by** a concentration of 100-400 mg/ml testosterone decanoate.

3. A pharmaceutical formulation according to claim 2, **characterized in that** the oily medium is castor oil.

4. The use of testosterone decanoate for the manufacture of a medicament in the treatment by injection of human androgen-insufficiency disorders, wherein testosterone decanoate is the sole androgen.

5. A use according to claim 4, wherein the testosterone decanoate is in the form of a formulation for injection of 300-700 mg to be given once per four to eight weeks.

6. A use according to claim 5, wherein the testosterone decanoate is to be given as 400 mg once every four weeks or 600 mg once every six weeks.

7. A drug delivery system for male contraception comprising a vehicle for the administration of a progestogen and a vehicle for the administration of an androgen, the vehicle for the progestogen containing etonogestrel and the vehicle for the androgen containing testosterone decanoate.

8. A drug delivery system according to claim 7, wherein the vehicle for etonogestrel is a subcutaneous implant, and the vehicle for testosterone decanoate is a subcutaneous injection fluid.

9. A drug delivery system according to claim 8, wherein the implant is of a type releasing 40 to 200 µg etonogestrel a day, preferably 120 to 180 µg a day.

10. A drug delivery system according to claim 8 or 9, wherein the injection fluid is a pharmaceutical formulation in accordance with claim 2.

11. A drug delivery system according to any one of claims 7 to 10 for the administration of testosterone decanoate in a regimen of 300 to 700 mg once every four to eight weeks, and preferably 400 mg once per four weeks or 600 mg once per six weeks.

## Patentansprüche

1. Verwendung eines Testosteron-Esters für die Herstellung eines Medikamentes zur Behandlung von menschlichen Androgen-Insuffizienz-Störungen, wobei das Medikament eine pharmazeutische Formulierung in der Form einer öligen Lösung zur Injektion umfassend Testosteron-Decanoat ist, welches in einem pharmazeutisch verträglichen öligen Medium aufgelöst ist, **dadurch gekennzeichnet, dass** das Testosteron-Decanoat der einzige Testosteron-Ester ist, der in dem öligen Medium vorhanden ist.

2. Pharmazeutische Formulierung nach Anspruch 1, **dadurch gekennzeichnet, dass** eine Konzentration von 100-400 mg/ml Testosteron-Decanoat vorhanden ist.

3. Pharmazeutische Formulierung nach Anspruch 2, **dadurch gekennzeichnet, dass** das ölige Medium Rizinusöl ist.

4. Verwendung des Testosteron-Decanoats zur Herstellung eines Medikamentes in der Behandlung durch Injektion von menschlichen Androgen-Insuffizienz-Störungen, wobei das Testosteron-Decanoat das einzige Androgen ist.

5. Verwendung nach Anspruch 4, bei der das Testosteron-Decanoat in Form einer Formulierung zur Injektion zwischen 300 bis 700 mg vorliegt, welches einmal je vier bis acht Wochen zu verabreichen ist.

6. Verwendung nach Anspruch 5, bei der das Testosteron-Decanoat als 400 mg jeweils alle vier Wochen oder 600 mg jeweils alle sechs Wochen zu verabreichen ist.

7. Medikamenten-Abgabesystem zur männlichen Verhütung umfassend ein Vehikel für die Verabreichung eines Progestogen und ein Vehikel für die Verabreichung eines Androgens, wobei das Vehikel für das Progestogen Etonogestrel enthält und das Vehikel für das Androgen Testosteron-Decanoat enthält.

8. Medikamenten-Abgabesystem nach Anspruch 7, bei dem das Vehikel für Etonogestrel ein subkutanes Implantat ist und bei dem das Vehikel für Testosteron-Decanoat eine subkutane Injektions-Flüssigkeit ist.

9. Medikamenten-Abgabesystem nach Anspruch 8, bei dem das Implantat von dem Typ ist, der täglich 40 bis 200 µg Etonogestrel abgibt, vorzugsweise 120 bis 180 µg pro Tag.

10. Medikamenten-Abgabesystem nach Anspruch 8 oder 9, bei dem die Injektions-Flüssigkeit eine pharmazeutische Formulierung in Übereinstimmung mit Anspruch 2 ist.

11. Medikamenten-Abgabesystem nach einem der Ansprüche 7 bis 10 für die Verabreichung von Testosteron-Decanoat in einem Regime zwischen 300 und 700 mg einmal jede vier bis acht Wochen und vorzugsweise 400 mg einmal je vier Wochen oder 600 mg einmal alle sechs Wochen.

## Revendications

1. Utilisation d'un ester de testostérone pour la fabrication de médicament pour le traitement des troubles de l'insuffisance androgénique humaine, où le médicament est une formulation pharmaceutique sous forme d'une solution huileuse pour injection comprenant du décanoate de testostérone dissous dans un milieu huileux pharmaceutiquement acceptable, **caractérisée en ce que** le décanoate de testostérone est le seul ester de testostérone présent dans le milieu huileux.

2. Une formulation pharmaceutique selon la revendication 1, **caractérisée en ce que** la concentration du décanoate de testostérone est de 100 à 400 mg/ml.

3. Une formulation pharmaceutique selon la revendication 2, **caractérisée en ce que** le milieu huileux est l'huile de ricin.

4. L'utilisation de décanoate de testostérone pour la fabrication d'un médicament destiné au traitement par injection des troubles de l'insuffisance androgénique humaine, où le décanoate de testostérone est le seul androgène.

5. Utilisation selon la revendication 4, dans laquelle le décanoate de testostérone est sous la forme d'une formulation injectable à 300 à 700 mg destinée à être administrée une fois tous les quatre à huit semaines.

6. Utilisation selon la revendication 5, dans laquelle le décanoate de testostérone doit être administré à raison de 400 mg une fois tous les quatre semaines ou 600 mg une fois toutes les huit semaines.

7. Un système de délivrance de médicament pour contraception masculine comprenant un véhicule pour l'administration d'un progestatif et un véhicule pour l'administration d'un androgène, le véhicule du progestatif contenant de l'étonogestrel et le véhicule de l'androgène contenant du décanoate de testostérone.

8. Un système de délivrance de médicament selon la revendication 7, dans lequel le véhicule pour l'étonogestrel est un implant sous-cutané et le véhicule pour le décanoate de testostérone est un fluide d'injection sous-cutanée.

9. Un système de délivrance de médicament selon la revendication 8, dans lequel l'implant est du type à libérer 40 à 200 µg d'étonogestrel par jour, de préférence 120 à 180 µg par jour.

10. Un système de délivrance de médicament selon la revendication 8 ou 9, dans lequel le fluide d'injection est une formulation pharmaceutique selon la revendication 2.

11. Un système de délivrance de médicament selon l'une quelconque des revendications 7 à 10 pour l'administration de décanoate de testostérone dans une posologie de 300 à 700 mg une fois toutes les quatre à huit semaines, et de préférence de 400 mg une fois toutes les quatre semaines ou 600 mg toutes les six semaines.
